# EUROPÄISCHE PATENTSCHRIFT

(11) **EP 2 934 620 B1**
(45) Veröffentlichungstag und Bekanntmachung des Hinweises auf die Patenterteilung: **14.12.2016**
(21) Anmeldenummer: 13818983.2
(22) Anmeldetag: 19.12.2013
(51) Int. Cl.: A61M 1/16, A61M 1/36

(54) **HÄMODIAFILTRATIONSVERFAHREN**
HAEMODIAFILTRATION METHOD
PROCÉDÉ D'HÉMODIAFILTRATION

(30) Priorität: 20.12.2012 DE 102012025052; 20.12.2012 US 201261740026 P
(43) Veröffentlichungstag der Anmeldung: 28.10.2015
(73) Patentinhaber: Fresenius Medical Care Deutschland GmbH, 61352 Bad Homburg (DE)
(72) Erfinder: TSCHULENA, Ulrich, 60439 Frankfurt/Main (DE); JANKOWSKI, Joachim, 52159 Roetgen (DE); FABIG, Anselm, 15738 Zeuthen (DE); MÜLLER, Carsten, 97502 Euerbach (DE)
(74) Vertreter: Behr, Wolfgang
(86) Internationale Anmeldenummer: PCT/EP2013/003870
(87) Internationale Veröffentlichungsnummer: WO 2014/095073

(56) Entgegenhaltungen:
- WO-A1-00/09182
- WO-A1-98/50091
- DE-A1-102010 028 902
- RO-B1- 122 077
- US-A1- 2005 015 040
- US-A1- 2005 082 225
- US-A1- 2009 221 948

## Beschreibung

Die vorliegende Erfindung betrifft eine Vorrichtung zur Durchführung einer Hämodiafiltrationsbehandlung unter Einwirkung eines elektromagnetischen Wechselfeldes und/oder eines elektrischen Gleichfeldes.

Die Aufgabe der gesunden Niere ist die Ausscheidung von Endprodukten des Stoffwechsels (harnpflichtige Substanzen) und Giftstoffen (Urämietoxine) aus dem Körper durch Bildung des Harns. Die Niere entfernen dabei ein breites Spektrum an Substanzen unterschiedlichen Molekulargewichts. Eine Übersicht über urämische Toxine wurde von R. Vanholder *et al.* veröffentlicht. (R. Vanholder et al., Kidney International, 63 (2003) 1934 - 1943) Die urämischen Toxine werden an Hand ihres Molekulargewichts in drei Klassen eingeteilt. Toxine mit einem Molekulargewicht von unter 500 Dalton bilden die Gruppe mit niedrigem Molekulargewicht. Die Mittelmoleküle liegen in einem mittleren Bereich mit einem Molekulargewicht zwischen 500 und 12 000 D. Zu den Mittelmolekülen gehört beispielsweise β₂-Microglobulin (11800 D). Die dritte Klasse von Urämietoxinen bilden Moleküle mit einem Molekulargewicht von über 12 000 D.

Darüber hinaus wird nach der Wasserlöslichkeit der Urämietoxine unterschieden. Beispiele für gut wasserlösliche urämische Toxine mit einem niedrigen Molekulargewicht sind Harnstoff, Creatinin, Oxalate, Guanidine und Harnsäure.

Beispiele für schlecht wasserlösliche urämische Toxine sind *p*-Cresol, Indoxylsulfat, Phenol, Hippursäure und Homocystein. Diese Urämietoxine liegen im Serum überwiegend an Proteinen gebunden vor.

Bei gesunden Personen werden die Urämietoxine über die Niere mit dem Harn ausgeschieden. Beim chronischen Nierenversagen verbleiben die urämischen Toxine jedoch im Blut des Patienten und müssen durch Hämodialyse oder Peritonealdialyse entfernt werden.

Während die Entfernung wasserlöslicher Toxine wie beispielsweise Harnstoff oder Creatinin mittels Hämodialyse sehr gut möglich ist, ist die Entfernung von schlecht wasserlöslichen hydrophoben Urämietoxinen mittels Hämodialyseverfahren durch die Proteinbindung stark erschwert. Man geht allgemein davon aus, daß ein chemisches Gleichgewicht zwischen dem freien, gelösten Toxin und dem proteingebundenen Toxin vorliegt, das weit auf Seiten des proteingebundenen Toxins liegt. Dies bedeutet, dass der überwiegende Teil dieser Urämietoxine an Proteine gebunden ist und nur ein kleiner Teil im Blutplasma gelöst ist.

Da es sich bei einem großen Teil der Substanzen um niedermolekulare Komponenten handelt, die zu einem geringen Teil in freier Form vorliegen, sind sie prinzipiell dialysierbar.

Weiterhin nimmt man an, daß Albumin als Bindungspartner der hydrophoben Urämietoxine fungiert. Albumin wird auf Grund seines Molekulargewichtes von Dialysemembranen zurückgehalten. Albumin wird durch Hämodialyseverfahren also nicht entfernt. Damit kann nur der freie, gelöste Anteil der urämischen Toxine aus dem Blut des Patienten entfernt werden. Die Einstellung des Gleichgewichtes unter der Dialyse wird zum geschwindigkeitsbestimmenden Schritt. Es ist zwar zu erwarten, dass sich nach der Entfernung der gelösten Toxine aus dem Blut das Gleichgewicht zwischen freien und proteingebundenen Toxinen wieder neu einstellt und bei genügend langer Dialysezeit ein beträchtlicher Teil der Toxine entfernen lässt, diese Zeit steht bei Hämodialysebehandlungen jedoch nicht zur Verfügung. Es besteht also ein Bedarf an Dialyseverfahren, die auch die proteingebundenen urämischen Toxine aus dem Blut des Patienten entfernen.

Die vorliegende Erfindung betrifft eine Vorrichtung zur Hämodiafiltration mit einem extrakorporalen Kreislauf zur Aufnahme von zu reinigendem Blut sowie mit einem Hämodialysator und/oder Hämofilter, der mit dem Blutkreislauf in Verbindung steht, wobei der Blutkreislauf stromaufwärts und stromabwärts von dem Hämodialysator und/oder Hämofilter jeweils mindestens eine Zuleitung für die Zufuhr einer Substitutionsflüssigkeit aufweist. Ferner weist die Vorrichtung ein Mittel zum Erzeugen eines hochfrequenten, elektromagnetischen Wechselfeldes und/oder eine Einrichtung zur Erzeugung einen elektrischen Gleichfeldes, wobei das zu reinigende Blut vor und/oder während des Kontaktes mit dem Dialysator dem hochfrequenten, elektromagnetischen Wechselfeld und/oder dem elektrischen Gleichfeld ausgesetzt wird. Die vorliegende Erfindung stellt damit ein Verfahren bereit, dass die Lage des Gleichgewichts zwischen freien und proteingebundenen Toxinen verschiebt und die Einstellung des Gleichgewichts während der Dialysebehandlung beschleunigt.

Verfahren zur Hämodialyse und zur Hämofiltration sind dem Fachmann bekannt. In den Veröffentlichungen "Replacement of Renal Function by Dialysis" (Drukker, Parsons und Maher; Kluwer Academic Publishers, 4. Auflage 1996; und "Hemodialysis Machines and Monitors" von H.-D. Polaschegg und N.W. Levin) - auf deren Offenbarung hiermit explizit verwiesen wird - findet sich eine Zusammenfassung der wichtigsten Hämodialyseverfahren und -maschinen: Bei der Hämodialyse wird das Blut eines Patienten durch eine arterielle Blutleitung in die Blutkammer eines Dialysators geleitet. Das Blut wird normalerweise mit Hilfe einer in der arteriellen Blutleitung angeordneten peristaltischen Rotationspumpe transportiert. Nach Durchgang durch die Pumpe wird das Blut durch die Blutkammer des Dialysators geleitet und schließlich durch eine venöse Tropfkammer und eine damit verbundene venöse Blutleitung zu dem Patienten zurückgeleitet. Ein Venendruckmonitor ist mit der venösen Tropfkammer als Schutzsystem zur unmittelbaren Erfassung eines Blutverlustes an die Umgebung verbunden. Gegebenenfalls können bei der sogenannten Einnadeldialyse zwei für die arterielle und die venöse Kanüle erforderliche Nadeln durch eine einzige Nadel ersetzt werden. Bei dieser Art der Dialyse besteht der extrakorporale Kreislauf aus einer Einnadelkanüle mit angeschlossenem Y-Stück. Von dem Dialysator führt die venöse Leitung zurück zu dem Y-Stück. Die arterielle und die venöse Leitung werden abwechselnd durch Klemmen verschlossen. Es laufen eine oder mehrere Blutpumpen, um für die abwechselnde Strömung zu und von dem Y-Stück zu sorgen.

Bei der Hämodialyse erfolgt die Entfernung der gelösten Stoffe aus dem Blut durch Diffusion durch die Dialysatormembran. Wenngleich zur Ultrafiltration des überschüssigen Wassers eines Patienten zusätzlich ein geringer transmembranöser Druck aufgebracht wird, spielt diese Filtration kaum eine Rolle für die Reinigung des Blutes von speziellen Substanzen.

Die Entfernung gelöster Stoffe bei der Hämofiltration erfolgt durch Konvektion und nicht durch Diffusion. Gleichzeitig wird das Ultrafiltrat fast vollständig durch eine Substitutionsflüssigkeit ersetzt, die eine ähnliche Zusammensetzung hat wie das Dialysat bei der Dialyse. Bei diesem Verfahren wird die Ahnlichkeit mit der natürlichen Niere und die wirksamere Entfernung größerer Moleküle betont. Die Entfernung niedermolekularer Substanzen ist dagegen gegenüber der Hämodialyse verringert, weil höchstens 45% des Blutes bei der sogenannten Nachverdünnungshämofiltration ultrafiltriert werden können. Heutzutage wird Hämofiltration wegen der hohen Kosten der handelsüblichen Austauschflüssigkeit und dem zur Durchführung der Behandlung innerhalb einer angemessenen Zeit erforderlichen hohen Blutdurchsatz nur bei einer kleinen Zahl von Patienten eingesetzt.

Hämofiltrationsmaschinen zur Dauerbehandlung umfassen dieselben extrakorporalen Pump- und Überwachungssysteme wie Hämodialysemaschinen. Der Dialysatkreislauf wird ersetzt durch ein Flüssigkeitsausgleichs- und - aufwärmsystem. Im sogenannten Vorverdünnungsmodus wird dem Blut stromaufwärts von dem Dialysator Substitutionsflüssigkeit zugesetzt, und das Filtrat wird durch den entsprechenden transmembranösen Druck erzeugt. Um klinisch wirksam zu sein, ist eine sehr große Menge Substitutionsflüssigkeit erforderlich. Wegen der hohen Kosten der handelsüblichen Substitutionsflüssigkeit hat sich dieses Verfahren bisher noch nicht durchgesetzt. üblicher ist der Nachverdünnungsmodus, weil weniger Substitutionsflüssigkeit erforderlich ist. In diesem Modus wird die Substitutionsflüssigkeit dem Blut stromabwärts von einem Dialysator zugesetzt. Im Nachverdünnungsmodus werden gute Reinigungskoeffizienten erzielt. Während einer 4-stündigen Behandlung werden normalerweise ungefähr 20 bis 24 Liter Substitutionsflüssigkeit zugesetzt. Die Wirksamkeit des Verfahrens ist jedoch durch einen kritischen transmembranösen Druck begrenzt, über dem es zu einer Schädigung des Blutes kommen wird.

Zum Flüssigkeitsausgleich wurden verschiedene Systeme vorgeschlagen. Bei dem gravimetrischen Ausgleichsverfahren kann Ultrafiltrat durch die Ultrafiltratpumpe in einen Beutel oder Behälter, der auf einer Ausgleichsplattform hängt oder steht, abgezogen werden. Substitutionsflüssigkeit aus einem Beutel oder Behälter auf derselben Plattform wid durch eine weitere Pumpe zu der venösen Tropfkammer gepumpt. Eine Nettoflüssigkeitsentnahme wird entweder durch eine zusätzliche Ultrafiltrationspumpe oder durch eine Programmiereinheit erreicht, die die Substitutionspumpe dahingehend steuert, dass sie weniger Flüssigkeit liefert als durch die Filtrationspumpe entfernt wurde.

Hämodiafiltration, eine Kombination aus Hämodialyse und Hämofiltration, kann durchgeführt werden, indem die extrakorporalen Kreisläufe einer Hämofiltrations- und einer Hämodialysemaschine kombiniert werden. Hämodialysemaschinen mit volumetrisch gesteuerter Ultrafiltration können leicht zurHämodiafiltration angepasst werden, die kostengünstiger ist. Diese ist besonders kostengünstig, wenn die Substitutionsflüssigkeit online aus der Dialyseflüssigkeit hergestellt wird.

Behandlungsparameter wie Dialysatgehalt (Natriumkonzentration), Ultrafiltrationsrate, Blut- und Dialysatdurchsatz werden während der Dialyse verändert, um die Wirksamkeit zu erhöhen oder aufrechtzuerhalten und/oder während der Dialyse auftretende Symptome zu verringern. Die Veränderung folgt entweder einem kinetischen Modell oder, häufiger, einer "klinischen Beurteilung". Während der Dialyse auftretende Symptome, insbesondere niedriger Blutdruck, stehen in engem Zusammenhang mit der Ultrafiltration. Bei Dialysemaschinen mit Ultrafiltrationspumpen, die von Dialysatpumpen unabhängig sind, erfolgt eine Profilierung durch Veränderung der Ultrafiltrationsgeschwindigkeit.

Zusammenfassend lässt sich sagen, dass bei der Hämodialyse das Blut des Patienten gereinigt wird, indem die zu entfernenden Substanzen des Blutes aufgrund eines Konzentrationsgefälles über die Membran des Dialysators durch die Membran diffundieren und dadurch die Dialyseflüssigkeit erreichen. Die treibende Kraft bei der Hämofiltration ist im Wesentlichen ein Druckunterschied über die Membran, der einen konvektiven Transport von Substanzen durch die Membran bewirkt und dabei das Blut vor allem auch von höhermolekularen Substanzen reinigt. Bei der Hämofiltration sowie bei dem kombinierten Verfahren der Hämodiafiltration wird Flüssigkeit aus dem Blut des Patienten entfernt, die bis auf einen kleinen Differenzbetrag zur Steuerung des Flüssigkeitsausgleichs substituiert werden muss.

Die Vorverdünnung wird vorzugsweise für Patienten verwendet, die ein höheres Risiko der Blutkoagulation bzw. Blutgerinnung haben. Dieses Risiko wird durch Verdünnung des Blutes vor der Blutbehandlung verringert.

Niedrige Hämatokritkonzentrationen führen zu entsprechend großen Mengen an freiem, d.h. ungebundenem Wasser, was einen charakteristischen konvektiven Transport von Substanzen durch die Membran möglich macht. Dementsprechend kann der Reinigungseffekt bei mittel- und hochmolekularen Substanzen im Vorverdünnungsmodus höher sein als im Nachverdünnungsmodus.

Darüber hinaus führt die Vorverdünnung des zu reinigenden Blutes dazu, dass mehr proteingebundene Urämietoxine in das Plasma übergehen und dialysiert werden können. Daher ist es bei der vorliegenden Erfindung vorteilhaft, wenn das Verhältnis der Infusionsgeschwindigkeiten (Qspre, Qspost) der Substitutionsflüssigkeit so gesteuert wird, dass Qspre stets größer oder gleich Qspost ist. Vorzugsweise beträgt das Verhältnis der Infusionsgeschwindigkeiten Qspre / Qspost mindestens 1,2 beträgt.

Um die Vorteile des Vor- und Nachverdünnungsmodus miteinander zu verbinden, wurde außerdem vorgeschlagen, beide Modi gleichzeitig mit einem festen Verhältnis des Durchsatzes an Vor- und Nachverdünnungssubstitutionsflüssigkeit anzuwenden (L. Pedrini und V. De Cristofaro, Abstract beim EDTNERA-Kongress in Madrid, 1999).

Das Dokument WO 98/50091 betrifft ein Verfahren zum Steuern einer Blutreinigungsvorrichtung, die stromaufwärts und stromabwärts von dem Filter jeweils mindestens eine Zuleitung zu dem Blutkreislauf zum Zuführen einer Substitutionsflüssigkeit umfasst. Eine Steuereinheit ist zum überwachen einer Blutpumpe, einer Ultrafiltratpumpe und der Substitutionsflüssigkeitspumpen sowie für Überwachungsmittel zum Abwiegen der entsprechenden Flüssigkeitsmenge vorgesehen. Die Steuereinheit überwacht die Pumpen in vorbestimmten Zeitabständen, um jeweils die momentane Strömungsgeschwindigkeit des Blutstroms, des Ultrafiltrats und der Substitutionsprodukte einzustellen.

Das Dokument WO 00/09182 betrifft eine Flüssigkeitsantriebsvorrichtung, die dazu geeignet ist, bestimmte Blutelemente und/oder Blutbestandteile durch Diffusion durch eine semipermeable Membran abzuziehen. Die Vorrichtung ist mit einer Blutpumpe, einer Pumpe zum Einspeisen von Vorverdünnungs-Substitutionsflüssigkeit, einer Pumpe zum Einspeisen von NachverdünnungsSubstitutionsflüssigkeit sowie einer Ultrafiltrationspumpe versehen. Ventile sind so angeordnet, dass die Flüssigkeit durch einen Behälter geleitet wird, der mit jeder der Pumpen in Flüssigkeitsverbindung gebracht werden kann, um die Funktionsweise der Pumpen und demzufolge die Strömungsgeschwindigkeiten der entsprechenden Flüssigkeiten zu steuern.

Ein weiterer Nachteil des Nachverdünnungsmodus besteht darin, dass sich während der Blutreinigung eine Begrenzungsmembran an der Membran des Hämodialysators und/oder Hämofilters aufbaut. Die Dicke dieser Membran nimmt mit zunehmender Dauer der Behandlung zu, was die Durchlässigkeit der Membran verringert. Dadurch wird - bei konstant bleibendem transmembranösem Druck - die Reinigungswirkung verschlechtert. Wenn eine konstante Reinigungswirkung erzielt werden soll, wäre ein zu nehmender transmembranöser Druck erforderlich, was zu einer Schädigung der Membran führen kann.

Das US-Patent 5,578,223 offenbart eine künstliche Niere, die in einem Nachverdünnungsmodus arbeitet und geeignet ist zur Verwendung bei einer Hämofiltrations-, Hämodialyse- und Hämodiafiltrationsbehandlung. Um eine gewünschte Bicarbonatkonzentration im Blut eines Patienten aufrechtzuerhalten, umfasst die Vorrichtung Mittel zum Perfundieren einer bicarbonathaltigen Flüssigkeit in den extrakorporalen Blutkreislauf nach Durchgang durch die Austausch- und Dosierungsmittel zum Einstellen der Bicarbonatkonzentration im Blut eines Patienten auf ein gewünschtes Niveau. Eine Extraktionspumpe, die mit dem Auslass des Austauschers verbunden ist, wird durch eine Steuereinheit gesteuert, um ein gewünschtes Maß an Gewichtsverlust während der Dauer der Behandlung zu erhalten. Die Strömungsgeschwindigkeit der Bicarbonatlösung wird durch die Steuereinheit in Abhängigkeit von der Strömungsgeschwindigkeit der Extraktionspumpe, der gewünschten Bicarbonatkonzentration im Blut eines Patienten und der Konzentration der Bicarbonatlösung vor Perfusion in den extrakorporalen Kreislauf gesteuert.

Aufgabe der vorliegenden Erfindung ist es, eine Vorrichtung zur Blutreinigung mittels Hämodialyse und/oder Hämofiltration bereitzustellen, mit der die Vorteile des Vorverdünnungsmodus und des Nachverdünnungsmodus kombiniert werden können und bei der gleichzeitig die Reinigungswirkung des Hämodialysators und/oder Hämofilters für proteingebundene Toxine verbessert wird.

Ausgehend von einer Vorrichtung gemäß dem Oberbegriff von Anspruch 1 wird diese Aufgabe dadurch gelöst, dass die Vorrichtung ferner Messvorrichtungen zum Aufzeichnen des transmembranösen Druckes und/oder Hämatokrits und/oder der Blutdichte umfasst, wobei die Messvorrichtungen mit einer Steuereinheit (100) zum Steuern eines oder mehrerer von transmembranösem Druck und/oder Hämatokrit und/oder Blutdichte verbunden sind, wobei die Steuereinheit so konstruiert ist, dass die Steuerung mit Hilfe mindestens einer der Infusionsgeschwindigkeiten der Substitutionsflüssigkeit durchgeführt wird und dass das zu reinigende Blut vor und/oder während des Kontaktes mit dem Dialysator einem hochfrequenten elektromagnetischen Feld und/oder elektrischen Gleichfeld ausgesetzt wird.

Die erfindungsgemäße Vorrichtung gemäß dem Oberbegriff von Anspruch 1 weist zusätzlich Mittel zur Erzeugung eines hochfrequenten elektromagnetischen Feldes und/oder eines elektrischen Gleichfeldes auf. Der Erfindung liegt die Erkenntnis zu Grunde, dass die Einstellung des Gleichgewichtes zwischen proteingebundenen und freien Toxinen mit Hilfe eines hochfrequenten elektromagnetischen Feldes und/oder eines elektrischen Gleichfeldes beschleunigt werden kann. Dem Fachmann sind solche Mittel bekannt. Die erfindungsgemäße Vorrichtung kann zur Erzeugung eines hochfrequenten, elektromagnetischen Feldes beispielsweise einen Hochfrequenzkondensator, eine Hochfrequenzspule und/oder eine Hochfrequenzelektrode aufweisen. Das hochfrequente elektromagnetische Feld weist eine Frequenz von 100 kHz bis 2 GHz, vorzugsweise 1 MHz bis 1 GHz, auf.

Weiterhin kann die erfindungsgemäße Vorrichtung Mittel zur Erzeugung eines elektrischen Gleichfeldes aufweisen. Dem Fachmann sind solche Mittel bekannt. Die erfindungsgemäße Vorrichtung kann beispielsweise aus einem Plattenkondensator mit zwei, vier oder mehr Platten aufgebaut sein. Das elektrische Gleichfeld weist eine Feldstärke bis zu 1500 V/m auf. In einer bevorzugten Ausführungsform weist das elektrische Gleichfeld eine Feldstärke von 10 V/m bis 400 V/m, besonders bevorzugt 100 V/m bis 250 V/m, auf. Durch niederfrequentes Umpolen der Kondensatorplatten kann ein rotierendes oder wanderndes Gleichfeld erzeugt werden.

Die Mittel zur Erzeugung eines hochfrequenten, elektromagnetischen Feldes und/oder eines elektrischen Gleichfeldes können derart ausgestaltet sein und im bzw. am Blutkreislauf angeordnet sein, dass das zu reinigende Blut dem hochfrequenten elektromagnetischen Feld ausgesetzt werden kann vor, während oder sowohl vor als auch während des Kontaktes des zu reinigenden Blutes mit dem Dialysator bzw. mit der semipermeablen Membran des Dialysators.

Durch Zugabe von Substitutionslösungen zu dem extrakorporalen Kreislauf stromaufwärts und stromabwärts von dem Hämodialysator und/oder Hämofilter können einerseits die Vorteile der Nachverdünnung und Vorverdünnung kombiniert werden, d.h. für niedermolekulare Substanzen sowie für mittel- und hochmolekulare Substanzen werden zufriedenstellende Reinigungsergebnisse erzielt. Andererseits werden gemäß der Erfindung die Infusionsgeschwindigkeiten einer oder beider stromaufwärts und stromabwärts zugeführten Substitutionsflüssigkeiten zur Steuerung von Betriebs- und/oder Blutparametern herangezogen.

Zum Beispiel im Falle eines hohen transmembranösen Druckes oder eines hohen Hämatokritwertes des Blutes kann also die Infusionsgeschwindigkeit der stromaufwärts von dem Dialysator zugegebenen Substitutionslösung erhöht werden, bis die gewünschten Werte für die zu steuernden Werte erreicht sind oder die Werte unter gegebene Grenzwerte fallen. Dementsprechend kann im Falle eines niedrigen transmembranösen Druckes oder eines niedrigen Hämatokritwertes die Infusionsgeschwindigkeit der stromabwärts von dem Dialysator zugeführten Substitutionsflüssigkeit erhöht werden, was infolge des dann resultierenden größeren Konzentrationsgefälles über die Membran zu einer Verbesserung des diffusiven Transports von Substanzen, d.h. zu einer verbesserten Reinigungswirkung für niedermolekulare Substanzen führt.

Die Infusionsgeschwindigkeit der stromaufwärts von dem Hämodialysator und/oder Hämofilter zugeführten Substitutionslösungen erhöht sich vorzugsweise gegenüber der stromabwärts von dem Hämodialysator und/oder Hämofilter zugeführten Infusionsgeschwindigkeit mit zunehmendem transmembranösem Druck und/oder zunehmender Blutdichte und/oder zunehmendem Hämatokritwert des Blutes.
Der transmembranöse Druck und/oder Hämatokrit und/oder die Blutdichte können kontinuierlich erfasst werden.

Besonders vorteilhaft ist es, wenn die Infusionsgeschwindigkeiten der Substitutionslösungen so gewählt sind, dass eine im Wesentlichen feststehende Begrenzungsmembran auf der der Kammer, durch die das Blut fließt, gegenüberliegenden Seite der Membran des Hämodialysators und/oder Hämofilters gebildet wird. Daraus resultiert der Vorteil, dass der Wirkungsgrad und das Spektrum des Siebkoeffizienten des Hämodialysators und/oder Hämofilters während der Zeit der Behandlung konstant bleiben.

Darüber hinaus führt die Vorverdünnung des zu reinigenden Blutes dazu, dass mehr proteingebundene Urämietoxine in das Plasma übergehen und dialysiert werden können - insbesondere durch den Einfluss des elektrischen Feldes. Daher ist es bei der vorliegenden Erfindung vorteilhaft, wenn das Verhältnis der Infusionsgeschwindigkeiten (Qspre, Qspost) der Substitutionsflüssigkeit so steuert, dass Qspre stets größer oder gleich Qspost ist. Vorzugsweise beträgt das Verhältnis der Infusionsgeschwindigkeiten Qspre / Qspost mindestens 1,2. Besonders bevorzugt beträgt das Verhältnis der Infusionsgeschwindigkeiten Qspre / Qspost mindestens 1,5.

Das Verhältnis der Infusionsgeschwindigkeiten der Substitutionslösungen Qspre / Qspost in dem Blutstrom kann nach Beendigung der Behandlung geändert werden, um die Begrenzungsmembran aufzulösen. Dadurch kann ein Großteil der die Begrenzungsmembran bildenden Proteine nach Beendigung der Blutbehandlung zu dem Patienten zurückgeleitet werden.

Die Messvorrichtungen können Drucksensoren umfassen, die jeweils in dem extrakorporalen Kreislauf und/oder in dem Dialyseflüssigkeitskreislauf stromaufwärts und/oder stromabwärts von dem Hämodialysator und/oder Hämofilter angeordnet sind.

Bei einer weiteren Ausführungsform der vorliegenden Erfindung umfassen die Messvorrichtungen Sensoren in dem extrakorporalen Kreislauf stromaufwärts und/oder stromabwärts von dem Hämodialysator und/oder Hämofilter zum Erfassen des Hämatokritwerts.

Gemäß einer bevorzugten Ausführungsform sind Mittel zum Steuern der mindestens einen der Infusionsgeschwindigkeiten (Qspre, Qspost) Pumpen in den Zuleitungen.

Bei einer weiteren Ausführungsform sind Mittel zum Steuern der mindestens einen der Infusionsgeschwindigkeiten (Qspre, Qspost) Ventile in den Zuleitungen.

Weitere Einzelheiten und Vorteile der vorliegenden Erfindung werden anhand der folgenden Figuren und Ausführungsbeispiele erläutert. In den Figuren zeigen:
- Figur 1:: eine schematische Darstellung eines Teils des extrakorporalen Kreislaufs sowie des Dialyseflüssigkeitskreislaufs mit Hämodialysator und Hämofilter sowie Zuleitungen für die Substitutionsflüssigkeit;
- Figur 2:: experimentelle Ergebnisse betreffend den Einfluss hochfrequenter elektromagnetischer Felder auf den proteingebundenen Anteil der Urämietoxine;
- Figur 3:: experimentelle Ergebnisse als Nachweis der fehlenden Beschädigung der Membran durch die hochfrequenten Felder;
- Figur 4:: experimentelle Ergebnisse betreffend die Einflüsse eines HF-Feldes im Frequenzbereich 1 bis 170 MHz auf den proteingebundenen Anteil der Urämietoxine;
- Figur 5:: experimentelle Ergebnisse betreffend die Einflüsse eines HF-Feldes im Frequenz-Bereich 110 bis 115 MHz auf den proteingebundenen Anteil der Urämietoxine;
- Figur 6:: experimentelle Ergebnisse betreffend die Einflüsse eines H-Feldes in den Frequenzbereichen 1 bis 6 MHz sowie 9 bis 13 MHz auf den proteingebundenen Anteil der Urämietoxine; und
- Figur 7:: experimentelle Ergebnisse betreffend die Einflüsse der Feldstärke auf den proteingebundenen Anteil der Urämietoxine.

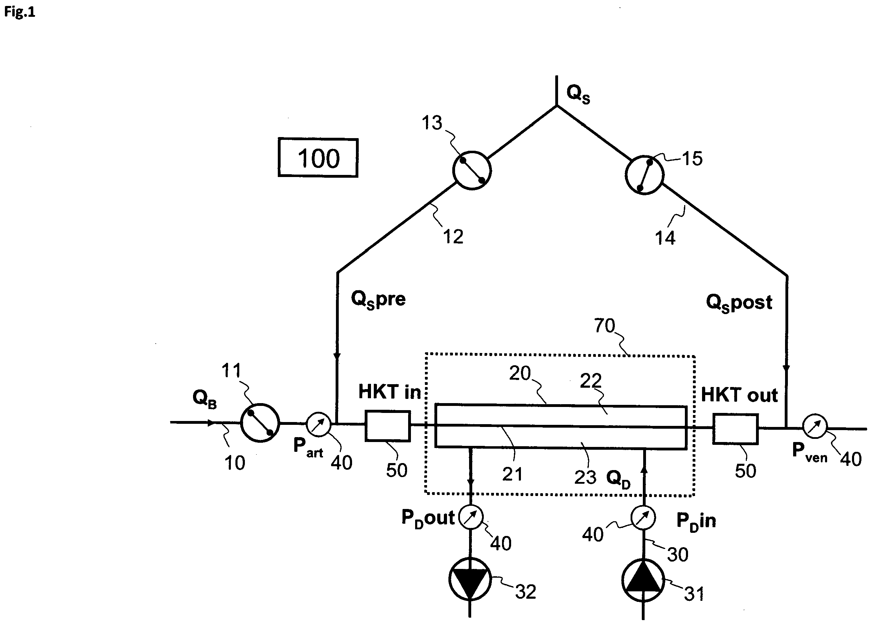

Figur 1 zeigt einen Teil des extrakorporalen Kreislaufs 10, durch den Blut mit der Strömungsgeschwindigkeit QB durch eine Blutpumpe 11 in Pfeilrichtung in Umlauf gebracht wird. In dem extrakorporalen Kreislauf 10 ist stromaufwärts von dem Hämodialysator oder Hämofilter 20 ein Drucksensor 40 sowie ein Sensor 50 zum Erfassen des arteriellen Blutdrucks Part sowie des Hämatokritwerts HKTin vor der Blutreinigung angeordnet.

Stromabwärts von dem Hämodialysator und/oder Hämofilter 20 sind entsprechende Messvorrichtungen 40, 50 zum Erfassen der entsprechenden Werte Pven und HKTout nach der Blutreinigung angeordnet.

Im Gegenstrom zum Blutstrom fließt Dialyseflüssigkeit in Pfeilrichtung mit der Strömungsgeschwindigkeit QD durch den Hämodialysator oder Hämofilter 20. Die Dialyseflüssigkeitsleitung 30 hat Drucksensoren 40 stromaufwärts und stromabwärts von dem Hämodialysator oder Hämofilter für den jeweiligen Druck PDin und PDout der Dialyseflüssigkeit. Die Zirkulation der Dialyseflüssigkeit wird durch Pumpen- und/oder Ausgleichsmittel 31 und 32 gesteuert.

Der Hämodialysator und/oder Hämofilter wird durch eine semipermeable Membran 21 in eine Blutkammer 22 und eine Dialyseflüssigkeitskammer 23 unterteilt.

Der Hämodialysator und/oder Hämofilter 20 wird von einem Mittel zur Erzeugung eines hochfrequenten elektromagnetischen Feldes und/oder eines elektrischen Gleichfeldes 70 umgeben.

In einer weiteren Ausführungsform wird neben dem Hämodialysator und/oder Hämofilter 20 auch ein davor stromaufwärts liegender Teil des extrakorporalen Blutkreislaufs 10 von einem Mittel zur Erzeugung eines hochfrequenten elektromagnetischen Feldes und/oder eines elektrischen Gleichfeldes 70 umgeben. Stromaufwärts und stromabwärts von dem Hämodialysator und/oder Hämofilter 20 sind Zuleitungen 12, 14 mit Flüssigkeitspumpen 13 bzw. 15 vorgesehen, mit denen Substitutionsflüssigkeit dem während der Behandlung in dem extrakorporalen Kreislauf 10 fließenden Blut zugeführt wird. Die jeweiligen Strömungsgeschwindigkeiten sind mit Qₛpre und Qₛpost gekennzeichnet.

Die beiden Infusionsgeschwindigkeiten Qₛpre und Qₛpost der Substitutionsflüssigkeit können gemäß der Erfindung mit Hilfe einer Steuereinheit 100 verändert werden. Die Steuereinheit 100 ist mit allen dargestellten Aktuatoren und Sensoren durch nicht dargestellte Verbindungen verbunden. Die Veränderung der Infusionsgeschwindigkeiten erfolgt gemäß den Messwerten der zu steuernden Steuerungswerte. Bei der in Figur 1 dargestellten Ausführungsform sind die Messwerte der arterielle und venöse Blutdruck Pₐᵣₜ, Pᵥₑₙ sowie der Druck der Dialyseflüssigkeit P_{D}in und P_{D}out vor und nach Durchgang durch den Hämodialysator und Hämofilter 20. Der daraus ermittelte transmembranöse Druck TMP wird gemäß der Erfindung durch eine geeignete Veränderung der Strömungsgeschwindigkeiten Qspre und Qspost auf den gewünschten Zielwert eingestellt oder wird auf diesem Wert gehalten. Anstelle des transmembranösen Druckes TMP können die Hämatokritwerte HKTᵢₙ, HKTₒᵤₜ als Steuerungswerte verwendet werden. TMP kann auch von weniger als den dargestellten vier Drucksensoren genähert werden. Bei den derzeit üblichen Dialysemaschinen werden Drucksensoren normalerweise nur für Pᵥₑₙ und P_{Dout} verwendet.

Mit Hilfe der beanspruchten Vorrichtung wird erreicht, dass die Begrenzungsmembran, die sich auf der der Kammer, in der das Blut vorhanden ist, gegenüberliegenden Seite der Membran des Hämodialysators oder Hämofilters aufbaut, in einem stationären Zustand gehalten werden kann, was in einem konstanten Reinigungsspektrum sowie einem konstanten Grad der Reinigung während der Behandlung resultiert. Gleichzeitig kann der transmembranöse Druck während der Behandlung konstant gehalten werden, da der durch die Membran und die Begrenzungsmembran bedingte Druckverlust ebenfalls konstant bleibt.

Durch die Begrenzung des transmembranösen Druckes auf einen vorbestimmbaren Wert kann die Gefahr eines durch große Konvektionskräfte bedingten extensiven Verlusts an Albumin durch die Membran verhindert werden. Bei Verwendung von Hochflussmembranen ist die Begrenzung des transmembranösen Druckes besonders wichtig.

Vor allem bei Patienten mit starken Gerinnungsproblemen trägt die Kombination aus Vor- und Nachverdünnung zur Verringerung des Verbrauchs an Heparin bei, das normalerweise in das Blut infundiert wird, um eine Blutgerinnung im extrakorporalen Kreislauf zu vermeiden. Wenn das Blut stromaufwärts von dem Hämodialysator und/oder Hämofilter verdünnt wird, ist weniger gerinnungshemmende Flüssigkeit erforderlich, um die Gefahr der Blutgerinnung in dem Hämodialysator und/oder Hämofilter zu verringern, da Letzterer das signifikanteste Potenzial zur Blutgerinnung in dem extrakorporalen Blutkreislauf dargestellt. Abgesehen von den oben genannten Vorteilen eines konstanten Betriebsverhaltens, kann durch die Kombination von Vorverdünnung und Nachverdünnung sowie durch die Einwirkung eines hochfrequenten elektromagnetischen Feldes und/oder eines elektrischen Gleichfeldes eine gute Reinigungsleistung für proteingebundene Urämietoxine erreicht werden.

Die nachfolgenden Versuchsergebnisse dienen als experimenteller Nachweis des Effektes eines elektrischen Feldes auf die Abtrennung proteingebundener Toxine während der Dialyse.

In Ausführungsbeispiel 1 wird der Effekt eines HF-Feldes im Frequenzbereich von 1 bis 20 MHz beschrieben. Ausführungsbeispiel 2 zeigt den Effekt des HF-Feldes im Frequenzbereich von 1 bis 170 MHz auf die Abtrennung von Phenylessigsäure. Unter dem Einfluss des HF-Feldes konnte die Abtrennrate für Phenylessigsäure um mindestens 45,3% gesteigert werden. Im Subband von 110 bis 120 MHz war der Effekt mit 54,6% besonders ausgeprägt. Im Ausführungsbeispiel 3 wird das Subband von 110 bis 120 MHz näher untersucht. Ausführungsbeispiel 4 zeigt den Einfluss eines H-Feldes in den Bereichen 1-6 MHz sowie 9-13 MHz. Ausführungsbeispiel 5 zeigt den Einfluss der Feldstärke auf die Abtrennung von Phenylessigsäure.

Die Temperatur wurde in allen Ausführungsbeispielen 1 bis 5 konstant gehalten, sodass die beobachteten Änderungen auf den Eigenschaften des elektrischen Feldes und nicht auf einer Erwärmung beruhen.

### Ausführungsbeispiel 1

In *in-vitro*-Versuchsreihen wurde der Einfluss hochfrequenter elektromagnetischer Felder auf den proteingebundenen Anteil der Urämietoxine untersucht.

Hierzu wurde ein Dialysemodul erstellt, indem konventionelle Hämofiltrations-Kapillaren mit Hilfe von Silikon als Schlaufen in einen Spritzenaufnahmestutzen eingegossen wurden. In das betreffende Modul wurde eine wässerige AlbuminLösung in Gegenwart der Urämie-Toxine Phenylessigsäure, p-Hydroxyhippursäure und Indoxylsulfat eingebracht. Mit Hilfe einer Spritzenpumpe wurde diese Lösung mit dem Dialysemodul für 10 min filtriert. Anschließend wurde durch Verwendung einer Hochfrequenz-Elektrode (HF-Elektrode) ein hochfrequentes elektromagnetisches Feld in der Lösung induziert. Das elektromagnetische Feld wird mittels einer Hochfrequenzspannungsquelle über einen Zeitraum von 10 Minuten von 1 bis 20 MHz in 1 MHz-Schritten inkrementiert. In den resultierenden Filtraten wurden die Konzentration der zuvor zum künstlichen Plasma gegebenen Urämie-Toxine Phenylessigsäure, p-Hydroxyhippursäure und Indoxylsulfat bestimmt. Durch Vergleich der Urämie-Toxin-Konzentrationen in den resultierenden Filtraten konnte der Effekt des HF-Feldes auf die Bindung zwischen Proteine und Urämie-Toxine evaluiert werden.

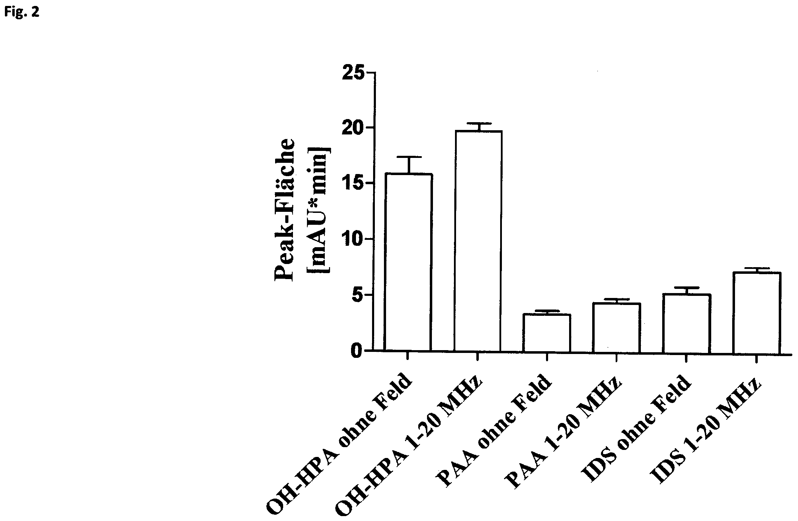

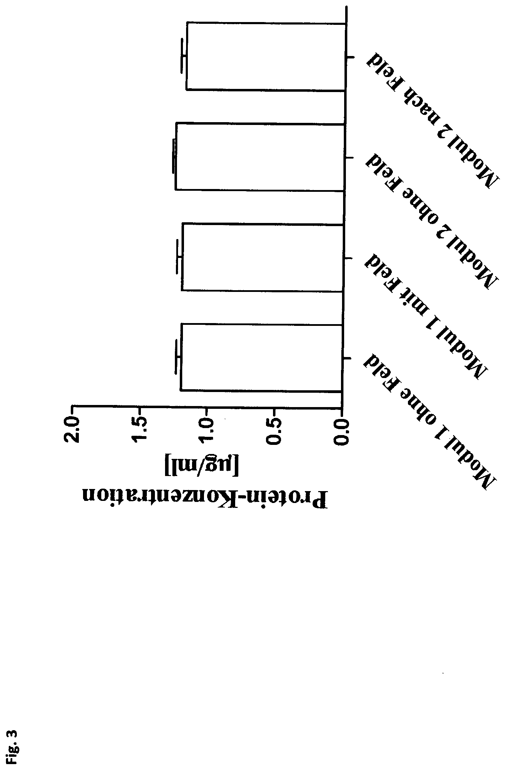

Die quantitative Bestimmung der Urämie-Toxin-Konzentration in den resultierenden Filtraten zeigte, dass hochfrequente elektromagnetische Felder die Filtrationsraten proteingebundener Urämie-Toxine signifikant erhöhen (Figur 2). Um zu überprüfen, ob hochfrequente elektromagnetische Felder die Dialysemembranen beschädigen, wurde die Proteinkonzentration im Filtrat mittels Proteinfärbung nach Bradford bestimmt. Die Ergebnisse zeigen, dass keine signifikanten Änderungen der Proteinkonzentration in Dialysemodulen ohne und unter Einfluss von hochfrequenten elektromagnetischen Feldern nachweisbar sind (Figur 3). Eine makroskopische Beschädigung der Membran ist aufgrund dieser Daten auszuschließen.

### Ausführungsbeispiel 2

Untersuchung der HF-Feldeinwirkung im Frequenz-Bereich 1 bis 170 MHz.

In das Dialysemodul aus Beispiel 1 wurde eine wässerige Lösung von bovinem Serumalbumin (BSA, 60 mg/ml) in Gegenwart des Urämie-Toxins Phenylessigsäure (1 mmol/l in 0.9 % NaCl-Lösung) eingebracht. Das HF-Feld wurde im Frequenz-Bereich 1-170 MHz in Subbändern von 10 MHz variiert und mit einem Kontrollversuch ohne HF-Feld verglichen.

Die quantitative Bestimmung der Phenylessigsäure erfolgte mittels HPLC.

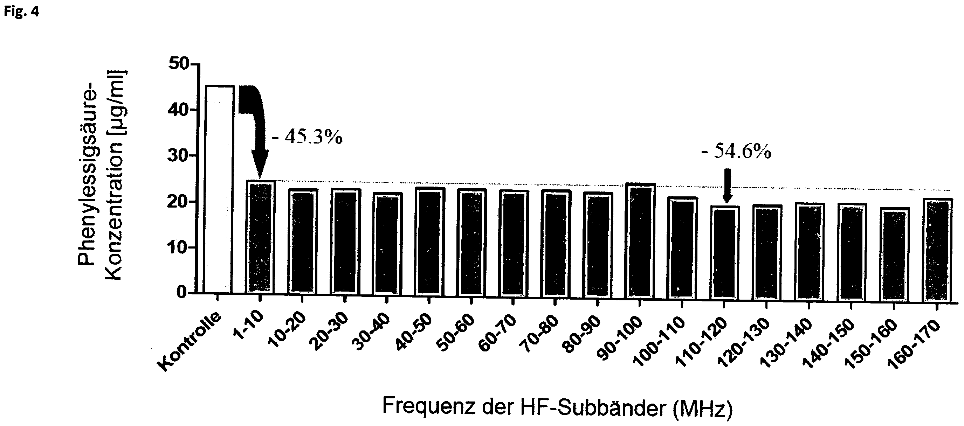

Die Ergebnisse der Versuche sind in Figur 4 zusammengefasst. Unter dem Einfluss des HF-Feldes konnte die Abtrennrate für Phenylessigsäure um mindestens 45,3% gesteigert werden. Im Subband von 110 bis 120 MHz war der Effekt mit 54,6% besonders ausgeprägt.

### Ausführungsbeispiel 3

Dieses Ausführungsbeispiel schließt an die Untersuchungen gemäß Ausführungsbeispiel 2 an, die gezeigt haben, dass der Effekt im Subband von 110 bis 120 MHz besonders ausgeprägt war.

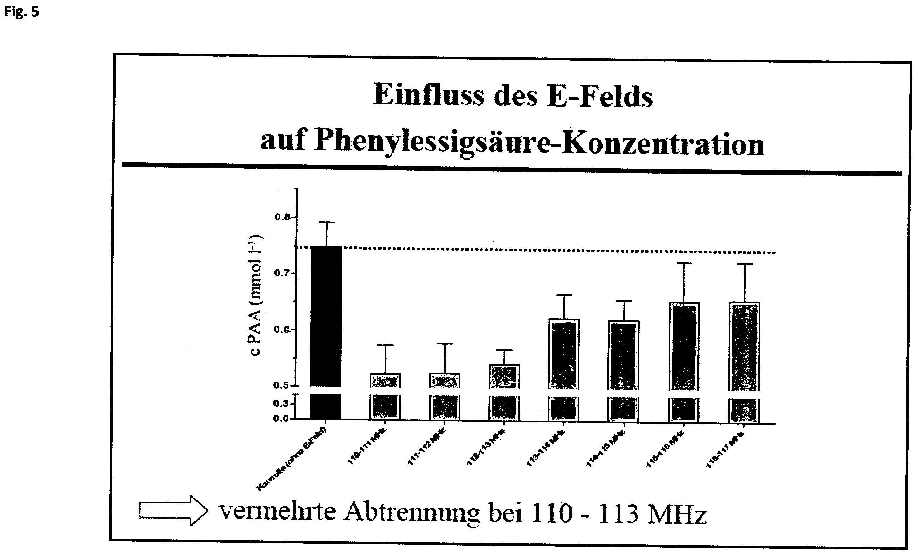

In den weiterführenden Untersuchungen gemäß Ausführungsbeispiel 3 konnte im Besonderen der Frequenzbereich um 110 bis 115 MHz als effektiver Frequenzbereich zur Freisetzung proteingebundener Urämietoxine identifiziert werden. Figur 5 zeigt den betreffenden Effekt auf die entsprechende Freisetzung und im Anschluss Abtrennung von Phenylessigsäure.

Nach bisherigem Stand eignen sich die im Folgenden in Tabelle 1 summarisch genannten Frequenzbereiche zur Abtrennung proteingebundener Urämietoxine.

**Tabelle 1: Geeignete Frequenzen im HF-Feld**

| **Frequenzen E-Feld** | **PAA** | **IDS** | **pCRS** |
|---|---|---|---|
| **80-120 MHz** | 110 | 110 | 110 |
| | 110-111 | 110-111 | 110-111 |
| | 111 | 111 | 111 |
| **120-170 MHz** | 140-141 | 140-141 | 140-141 |
| | 148-149 | | 151-152 |
| | 160-161 | | |

Bei den betreffenden Frequenzbereichen handelt es sich um die Bereiche, bei denen der maximale Abtrenneffekt bestimmt worden ist. In den nicht-genannten Frequenzbereichen wurde teilweise eine im Vergleich zu Kontrolle vermehrte Abtrennung bestimmt, die jedoch geringer war, als in den genannten Frequenzbereichen.

### Ausführungsbeispiel 4:

Weiterhin konnte auch im Bereich des H-Feld eine vermehrte Freisetzung und damit Abtrennung der proteingebundener Urämietoxine bestimmt werden.

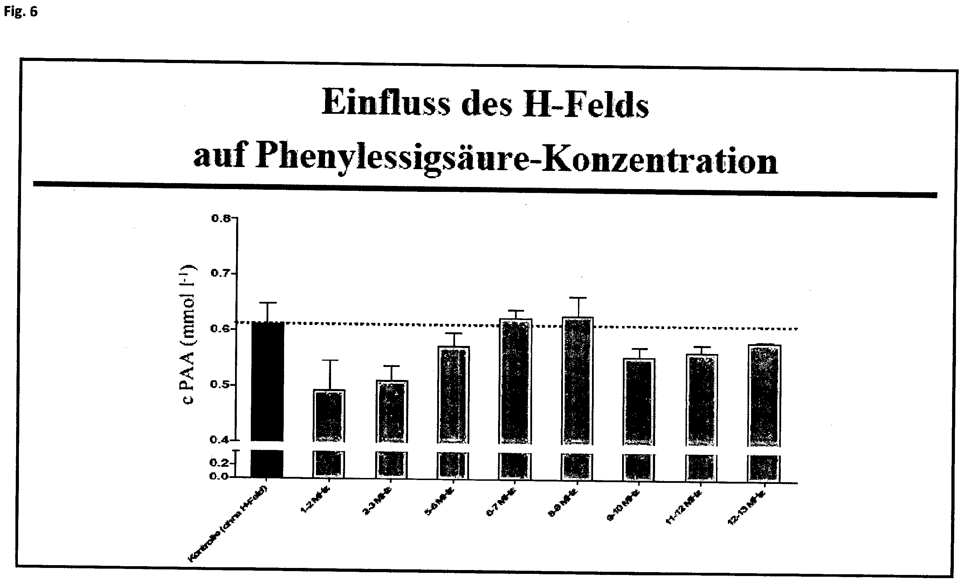

Figur 6 ist zu entnehmen, dass der H-Feld-Bereich von 1-6 MHz sowie der Bereich 9-13 MHz geeignet ist, proteingebundener Urämietoxine aus der Proteinbindung freizusetzen und in Folge dialytisch abzutrennen. Gezeigt ist in Figur 6 der Effekt auf Phenylessigsäure.

### Ausführungsbeispiel 5:

Neben der Frequenz des eingesetzten Feldes ist auch dessen Feldstärke für die resultierende Freisetzung und Abtrennung relevant. Mit zunehmender Feldstärke werden vermehrt die betreffenden Urämietoxine aus der Proteinbindung freigesetzt und anschließend abtrennt werden.

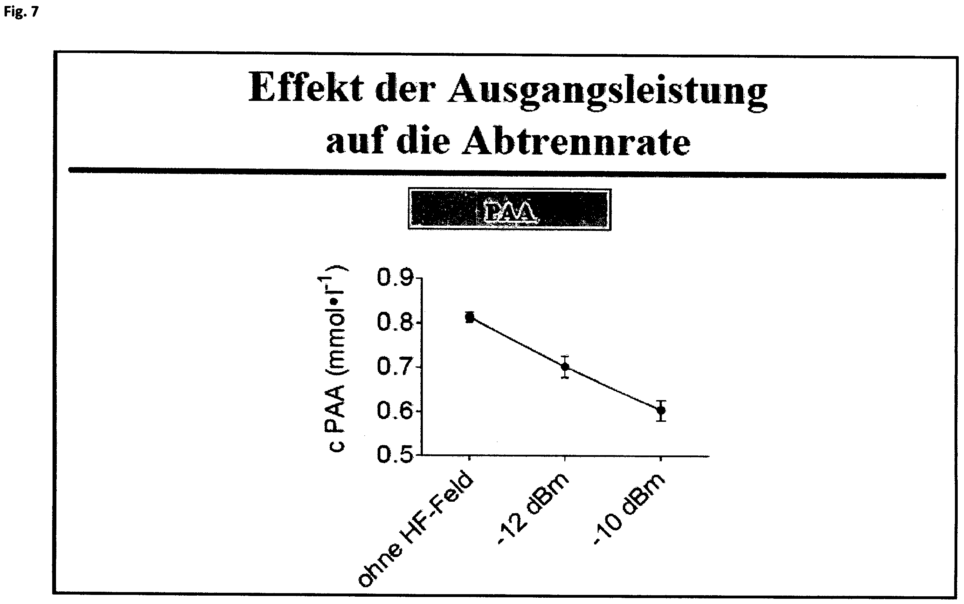

Figur 7 zeigt den Effekt einer ansteigenden Feldstärke auf den Gehalt proteingebundener Urämietoxine im Retentat am Beispiel der Phenylessigsäure.

## Patentansprüche

1. Vorrichtung zur Hämodiafiltration mit einem extrakorporalen Kreislauf (10) zur Aufnahme von zu reinigendem Blut sowie mit einem Hämodialysator und/oder Hämofilter (20), der mit dem Blutkreislauf (10) in Verbindung steht, wobei der Blutkreislauf (10) stromaufwärts und stromabwärts von dem Hämodialysator und/oder Hämofilter (20) jeweils mindestens eine Zuleitung (12, 14) für die Zufuhr einer Substitutionsflüssigkeit aufweist, **dadurch gekennzeichnet, dass** die Vorrichtung ferner Messvorrichtungen zum Aufzeichnen des transmembranösen Druckes und/oder Hämatokrits (HKT) und/oder der Blutdichte umfasst, wobei die Messvorrichtungen mit einer Steuereinheit (100) zum Steuern eines oder mehrerer von transmembranösem Druck und/oder Hämatokrit (HKT) und/oder Blutdichte verbunden sind, wobei die Steuereinheit (100) so konstruiert ist, dass die Steuerung mit Hilfe mindestens einer der Infusionsgeschwindigkeiten (Qₛpre, Qₛpost) der Substitutionsflüssigkeit durchgeführt wird (13, 15) und dass das zu reinigende Blut vor und/oder während des Kontaktes mit dem Hämodialysator und/oder Hämofilter (20) einem hochfrequenten elektromagnetischen Feld und/oder einem elektrischen Gleichfeld (70) ausgesetzt wird.

2. Vorrichtung nach Anspruch 1, **dadurch gekennzeichnet, dass** die Messvorrichtungen Sensoren (40) umfassen, die in dem extrakorporalen Kreislauf (10) und/oder in dem Dialyseflüssigkeitskreislauf (30) stromaufwärts und/oder stromabwärts von dem Hämodialysator und/oder Hämofilter (20) angeordnet sind.

3. Vorrichtung nach einem der vorhergehenden Ansprüche wobei die Steuereinheit (100) die Infusionsgeschwindigkeiten (Qₛpre, Qₛpost) der Substitutionsflüssigkeit so steuert, dass Qₛpre während der Behandlung größer oder gleich Qₛpost ist.

4. Vorrichtung nach Anspruch 3, wobei das Verhältnis der Infusionsgeschwindigkeiten Qₛpre / Qₛpost mindestens 1,2 beträgt.

5. Vorrichtung nach Anspruch 3 oder 4, wobei das Verhältnis der Infusionsgeschwindigkeiten Qₛpre / Qₛpost mindestens 1,5 beträgt.

6. Vorrichtung nach einem der vorhergehenden Ansprüche, wobei das hochfrequente elektromagnetische Feld von einer Hochfrequenzspule, einer Hochfrequenzelektrode und/oder einem Hochfrequenzkondensator erzeugt wird.

7. Vorrichtung nach einem der vorhergehenden Ansprüche, wobei das elektrische Gleichfeld von einem Kondensator mit mindestens zwei Platten erzeugt wird.

8. Vorrichtung nach einem der vorhergehenden Ansprüche, wobei das hochfrequente elektromagnetische Feld eine Frequenz von 100 kHz bis 2 GHz, vorzugsweise 1 MHz bis 1 GHz, aufweist.

9. Vorrichtung nach einem der vorhergehenden Ansprüche, wobei das elektrische Gleichfeld eine Feldstärke bis zu 1500 V/m aufweist

10. Vorrichtung nach einem der vorhergehenden Ansprüche, wobei das elektrische Gleichfeld eine Feldstärke von 10 bis 400 V/m, aufweist.

## Claims

1. An apparatus for hemodiafiltration with an extracorporeal circulation (10) for receiving blood to be purified and having a hemodialyzer and/or hemofilter (20) which is connected to the blood circulation (10), wherein the blood circulation (10) has at least one inlet line (12, 14) for the supply of a replacement fluid upstream and downstream from the hemodialyzer and/or hemofilter (20),
**characterized in that**
the apparatus also comprises measurement apparatuses for recording the transmembrane pressure and/or hematocrit (HKT) and/or blood density, wherein the measurement apparatuses are connected to a control unit (100) for controlling one or more of the transmembrane pressure and/or the hematocrit (HKT) and/or the blood density, wherein the control unit (100) is constructed so that the control is implemented with the help of at least one of the infusion rates (Qₛpre, Qₛpost) of the replacement fluid (13, 15), and the blood to be purified is exposed to a high-frequency electromagnetic field and/or an electric DC field (70) before and/or during contact with the hemodialyzer and/or hemofilter (20).

2. The apparatus according to Claim 1,
**characterized in that**
the measurement apparatuses comprise sensors (40) which are arranged in the extracorporeal circulation (10) and/or in the dialysis fluid circulation (30) upstream and/or downstream from the hemodialyzer and/or hemofilter (20).

3. The apparatus according to any one of the preceding claims, wherein the control unit (100) controls the infusion rates (Qₛpre, Qₛpost) of the replacement fluid so that Qₛpre is greater than or equal to Qₛpost during the treatment.

4. The apparatus according to Claim 3,
wherein the ratio of the infusion rates Qₛpre/Qₛpost is at least 1.2.

5. The apparatus according to Claim 3 or 4,
wherein the ratio of the infusion rates Qₛpre/Qₛpost is at least 1.5.

6. The apparatus according to any one of the preceding claims,
wherein the high-frequency electromagnetic field is generated by a high-frequency coil, a high-frequency electrode and/or a high-frequency capacitor.

7. The apparatus according to any one of the preceding claims,
wherein the electric DC field is generated by a capacitor having at least two plates.

8. The apparatus according to any one of the preceding claims,
wherein the high-frequency electromagnetic field has a frequency of 100 kHz to 2 GHz, preferably 1 MHz to 1 GHz.

9. The apparatus according to any one of the preceding claims,
wherein the electric DC field has a field strength of up to 1500 V/m.

10. The apparatus according to any one of the preceding claims,
wherein the electric DC field has a field strength of 10 to 400 V/m.

## Revendications

1. Dispositif d'hémodiafiltration comprenant un circuit extracorporel (10) destiné à recevoir du sang à nettoyer ainsi qu'un hémodialyseur et/ou hémofiltre (20), qui est en liaison avec le circuit sanguin (10), le circuit sanguin (10) comportant en amont et en aval de l'hémodialyseur et/ou de l'hémofiltre (20) respectivement au moins une conduite d'amenée (12, 14) pour l'alimentation d'un liquide de substitution, **caractérisé en ce que** le dispositif comprend en outre des dispositifs de mesure destinés à enregistrer la pression transmembranaire et/ou l'hématocrite (HKT) et/ou la densité du sang, les dispositifs de mesure étant reliés à une unité de commande (100) destinée à la commande de la pression transmembranaire et/ou l'hématocrite (HKT) et/ou la densité du sang, l'unité de commande (100) étant conçue de telle manière que la commande est exécutée (13, 15) à l'aide d'au moins une des vitesses de perfusion (Qₛpre, Qₛpost) du liquide de substitution et que le sang à nettoyer est exposé avant et/ou pendant le contact avec l'hémodialyseur et/ou l'hémofiltre (20) à un champ électromagnétique haute fréquence et/ou à un champ de courant continu (70).

2. Dispositif selon la revendication 1, **caractérisé en ce que** les dispositifs de mesure comprennent des capteurs (40), qui sont disposés dans le circuit extracorporel (10) et/ou dans le circuit de liquide de dialyse (30) en amont et/ou en aval de l'hémodialyseur et/ou de l'hémofiltre (20).

3. Dispositif selon l'une des revendications précédentes, dans lequel l'unité de commande (100) commande les vitesses de perfusion (Qₛpre, Qₛpost) du liquide de substitution de telle manière que, pendant le traitement, Qₛpre est supérieure ou égale à Qₛpost.

4. Dispositif selon la revendication 3, dans lequel le rapport des vitesses de perfusion Qₛpre / Qₛpost est d'au moins 1,2.

5. Dispositif selon la revendication 3 ou 4, dans lequel le rapport des vitesses de perfusion Qₛpre / Qₛpost est d'au moins 1,5.

6. Dispositif selon l'une des revendications précédentes, dans lequel le champ électromagnétique haute fréquence est généré par une bobine à haute fréquence, une électrode à haute fréquence et/ou un condensateur à haute fréquence.

7. Dispositif selon l'une des revendications précédentes, dans lequel le champ de courant continu est généré par un condensateur comprenant au moins deux plaques.

8. Dispositif selon l'une des revendications précédentes, dans lequel le champ électromagnétique haute fréquence présente une fréquence de 100 kHz à 2 GHz, de préférence de 1 MHz à 1 GHz.

9. Dispositif selon l'une des revendications précédentes, dans lequel le champ de courant continu présente une intensité du champ allant jusqu'à 1500 V/m.

10. Dispositif selon l'une des revendications précédentes, dans lequel le champ de courant continu présente une intensité du champ de 10 à 400 V/m.
